# EUROPEAN PATENT APPLICATION

(11) **EP 3 342 452 A1**
(43) Date of publication of application: **04.07.2018**
(21) Application number: 17305004.8
(22) Date of filing: 03.01.2017
(51) Int. Cl.: A61N 1/04, A61K 8/49, A61N 1/30, A61N 1/32, A61K 31/366

(54) **SYSTEMS INCLUDING ASSOCIATION OF ELLAGIC ACID AND MICROCURRENT**

(71) Applicant: L'OREAL, 75008 Paris (FR)
(72) Inventor: CAZARES DELGADILLO, Jennyfer, 94152 Chevilly Larue (FR); PLANARD-LUONG, Thi Hong Lien, 94152 Chevilly Larue (FR)
(74) Representative: Nony

(57) **Abstract**

lontophoretic administration of ellagic acid or derivatives including: applying a potential to a device having a first electrode where ellagic acid or derivatives are to be administered and a second electrode, wherein the ellagic acid composition includes from 0.01% by weight to 30% by weight of ellagic acid or ellagic acid derivatives or their ions, from 1 % by weight to 99 % by weight of polar solvents, including water. Then, conducting electrical current between the first and second electrodes, wherein the current passes through the ellagic acid composition such that a polarity and magnitude of current density is sufficient to transport ellagic acid ions or ions or both at least into the skin. The current density profiles include constant direct current, pulse current, and direct current in combination with pulse current. In an embodiment, the Ellagic acid compositions are formulated to be used with the various waveforms.

## Description

### SUMMARY

In an aspect, the present disclosure is directed to, among other things, an iontophoresis device including an electrode assembly having at least one electrode and an ellagic acid composition. In an embodiment, the ellagic acid composition includes one or more of ellagic acid, an ellagic acid derivative, an ion of ellagic acid, and an ion of an ellagic acid derivative. In an embodiment, the electrode assembly includes at least one reservoir holding an ellagic acid composition comprising one or more of ellagic acid, an ellagic acid derivative, an ion of ellagic acid, and an ion of an ellagic acid derivative present in amounts ranging from about 0.01 % to about 30% by weight; and a polar solvent present in an amount ranging from about 0.1 % by weight to about 95% by weight. In an embodiment, the iontophoresis device includes circuitry operably coupled to the electrode assembly and configured to concurrently generate at least a continuous direct current stimulus and a pulsed current stimulus at a first electrode. In an embodiment, the continuous direct current stimulus and the pulsed current stimulus are of a character and for a duration sufficient to transdermally deliver an ellagic acid composition to a biological subject. In an embodiment, the device is configured to electrochemically deliver an ellagic acid through the skin by applying a DC, unidirectional current. In an embodiment, the device includes circuitry configured to apply a unidirectional direct current of a character and for a duration sufficient to deliver an ellagic acid through the skin.

In an aspect, the present disclosure is directed to, among other things, an iontophoresis method including concurrently delivering a continuous direct current and a pulsed current to a biological subject, the continuous direct current and the pulsed current of a character and for a duration sufficient to transdermally deliver an ellagic acid composition including at least one of ellagic acid, an ellagic acid derivative, an ion of ellagic acid, an ion of an ellagic acid derivative, or mixtures thereof. In an embodiment, the iontophoresis method includes transdermally delivering an ellagic acid composition including one or more of ellagic acid, an ellagic acid derivative, an ion of ellagic acid, and an ion of an ellagic acid derivative present in amounts ranging from about 0.01% to about 30% by weight; and a polar solvent present in an amount ranging from about 0.1 % by weight to about 99 % by weight.

In an aspect, the present disclosure is directed to, among other things, an iontophoresis composition including one or more of ellagic acid, an ellagic acid derivative, an ion of ellagic acid, and an ion of an ellagic acid derivative present in amounts ranging from about 0.01% to about 30% by weight; and a polar solvent present in an amount ranging from about 0.1% by weight to about 99% by weight. In an embodiment, the iontophoresis composition comprises a pH ranging from about 4 to about 7.4.

Many different aspects and embodiments are possible. Some of those aspects and embodiments are described below. After reading this specification, skilled artisans will appreciate that those aspects and embodiments are only illustrative and do not limit the scope of the present invention. Embodiments may be in accordance with any one or more of the items as listed below.
item 1. An iontophoresis device, comprising:
   a. an electrode assembly including at least one electrode and an ellagic acid composition, the ellagic acid composition including one or more of ellagic acid, an ellagic acid derivative, an ion of ellagic acid, and an ion of an ellagic acid derivative; and
   b. circuitry operably coupled to the electrode assembly and configured to concurrently generate at least a continuous direct current stimulus and a pulsed current stimulus at a first electrode, the continuous direct current stimulus and the pulsed current stimulus of a character and for a duration sufficient to transdermally deliver the ellagic acid composition to a biological subject.
item 2. The iontophoresis device of item 1, wherein the electrode assembly includes at least one reservoir holding an ellagic acid composition comprising
   a. one or more of ellagic acid, an ellagic acid derivative, an ion of ellagic acid, and an ion of an ellagic acid derivative present in amounts ranging from about 0.01% to about 30% by weight; and
   b. a polar solvent present in an amount ranging from about 0.1 % by weight to about 99% by weight.
item 3. The iontophoresis device of item 2, wherein the electrode assembly includes at least one reservoir holding an ellagic acid composition further comprising one or more of
   a. Acrylates/C10-30 alkyl acrylate crosspolymer present in an amount ranging from about 0% by weight to about 1% by weight;
   b. Cetyl alcohol present in an amount ranging from about 0 % by weight to about 1% by weight;
   c. Deionized water present in an amount ranging from about 20% by weight to about 60% by weight;
   d. Diisopropyl sebacate present in an amount ranging from about 0 % by weight to about 9% by weight;
   e. Dimethicone present in an amount ranging from about 0% by weight to about 5% by weight;
   f. Ethylparaben present in an amount ranging from about 0% by weight to about 1% by weight;
   g. Fragrance present in an amount ranging from about 0 % by weight to about 1% by weight;
   h. Ginkgo Biloba extract present in an amount ranging from about 0% by weight to about 1% by weight;
   i. Glycerin present in an amount ranging from about 0% by weight to about 14% by weight;
   j. Glyceryl Stearate and polyethylene glycol-100 stearate present in an amount ranging from about 0% by weight to about 3% by weight;
   k. Glycyrrhiza Glabra root extract present in an amount ranging from about 0% by weight to about 1% by weight;
   1. Helianthus Annuus seed extract present in an amount ranging from about 0% by weight to about 1 % by weight;
   m. Hydrolyzed soy flour present in an amount ranging from about 0% by weight to about 1% by weight;
   n. Mentha Piperita extract present in an amount ranging from about 0% by weight to about 1% by weight;
   o. Methyl gluceth-20 present in an amount ranging from about 0% by weight to about 6% by weight;
   p. Methylparaben present in an amount ranging from about 0% by weight to about 1% by weight;
   q. Octyldodecanol present in an amount ranging from about 0% by weight to about 4% by weight;
   r. Phenoxyethanol present in an amount ranging from about 0% by weight to about 1% by weight;
   s. Polyacrylamide and C13-14 isoparaffin and laureth-7 present in an amount ranging from about 0% by weight to about 2% by weight;
   t. Polypropylene glycol-5-ceteth-20 present in an amount ranging from about 0% by weight to about 1 % by weight;
   u. Propylene glycol present in an amount ranging from about 0% by weight to about 20% by weight;
   v. Rosa Centifolia flower extract present in an amount ranging from about 0% by weight to about 1% by weight;
   w. Salicylic acid present in an amount ranging from about 0% by weight to about 1% by weight;
   x. Sodium Hydroxide present in an amount ranging from about 0% by weight to about 1% by weight;
   y. Stearic acid present in an amount ranging from about 0% by weight to about 2% by weight;
   z. Tetrasodium ethylenediaminetetraacetic acid present in an amount ranging from about 0% by weight to about 1% by weight;
   aa. Tocopherol present in an amount ranging from about 0% by weight to about 1% by weight;
   bb. Xanthan gum present in an amount ranging from about 0% by weight to about 1% by weight; and
   cc. Yeast extract present in an amount ranging from about 0% by weight to about 1% by weight.
item 4. The iontophoresis device of item 1, wherein the continuous direct current stimulus comprises an average current density raging from about 0.01 mA/cm² to about 0.5 mA/cm².
item 5. The iontophoresis device of item 1, wherein the continuous direct current stimulus comprises an average current density of about 0.2 mA/cm².
item 6. The iontophoresis device of item 1, wherein the pulsed current stimulus comprises an average current density raging from about 0.01 mA/cm² to about 10 mA/cm²; a pulse width ranging from about 50 microseconds to about 100 milliseconds; and a pulse frequency ranging from about 1 Hertz to about 2000 Hertz; and the duty cycle of pulses ranging from about 1% to about 90%.
item 7. The iontophoresis device of item 1, wherein the pulsed current stimulus comprises an average current density raging from about 0.01 mA/cm² to about 10 mA/cm²; a pulse width ranging from about 50 microseconds to about 100 milliseconds; at least one pulse train having from 2 to 20 pulses; a pulse frequency ranging from about 0.1 Hertz to about 500 Hertz; and a duty cycle ranging from 1% to 90%.
item 8. The iontophoresis device of item 1, wherein the pulsed current stimulus comprises an average current density raging from about 0.01 mA/cm² to about 10 mA/cm²; a pulse width ranging from about 50 microseconds to about 100 milliseconds; and at least one pulse train of about 2 to about 20 pulses with alternating polarity; a pulse frequency ranging from about 0.1 Hertz to about 500 Hertz; and a duty cycle of pulses ranging from about 1% to about 90%.
item 9. The iontophoresis device of item 1, wherein the pulsed current stimulus comprises an amplitude current density of about 0.2 mA/cm²; an alternating pulse duration of about 500 microseconds; and a pulse frequency of about 200 Hertz.
item 10. The iontophoresis device of item 1, wherein the electrode assembly includes at least one reservoir holding an ellagic acid composition.
item 11. The iontophoresis device of item 1, wherein the electrode assembly includes at least one active electrode electrically coupled to a reservoir holding an ellagic acid composition; the electrode assembly operable to transdermally deliver the ellagic acid composition to a biological subject responsive to one or more inputs from the circuitry configured to concurrently generate the continuous direct current stimulus and the pulsed current stimulus.
item 12. The iontophoresis device of item 1, wherein the electrode assembly is electrically coupled to at least one power source.
item 13. The iontophoresis device of item 1, wherein the electrode assembly includes a least one active electrode assembly and at least one counter electrode assembly.
item 14. An iontophoresis assembly, comprising:
   a. a current waveform generator with circuitry configured to
   b. apply direct current at a current density of at most 0.5 mA/cm2 for up to 30 minutes, or
   c. apply a pulse waveform current density at an amplitude of at most 1 mA/cm² for up to 30 minutes, or
   d. apply concurrently a direct current waveform and a pulse waveform at an average current density of at most 0.5 mA/cm2 for up to 30 minutes, or a combination thereof in sequential with or without period of pause of up to 120 minutes.
item 15. The iontophoresis assembly of item 9, wherein the pulse waveform includes monophasic pulses having a constant slope between a maximum and a minimum amplitude, or includes biphasic pulses having a constant slope between a maximum amplitude in a first current direction and a maximum amplitude in a second current direction.
item 16. The iontophoresis assembly of item 9" further comprising an ellagic acid composition within the device, wherein the ellagic composition comprises: from 0.01% by weight to 30% by weight ellagic acid or ellagic acid derivatives or their ions, and from 0.1 % by weight to 99% by weight of organic polar solvents, including water.
item 17. An iontophoresis method, comprising:
   a. concurrently delivering a continuous direct current and a pulsed current to a biological subject, the continuous direct current and the pulsed current of a character and for a duration sufficient to transdermally deliver an ellagic acid composition including at least one of ellagic acid, an ellagic acid derivative, an ion of ellagic acid, an ion of an ellagic acid derivative, or mixtures thereof.
item 18. The iontophoresis method of item 10, wherein concurrently delivering the continuous direct current and the pulsed current to a biological subject includes generating a continuous direct current stimulus having an average current density raging from about 0.01 mA/cm² to about 0.5 mA/cm².
item 19. The iontophoresis method of item 10, wherein concurrently delivering the continuous direct current and the pulsed current to a biological subject includes generating a continuous direct current stimulus having an average current density of about 0.2 mA/cm².
item 20. The iontophoresis method of item 10, wherein concurrently delivering the continuous direct current and the pulsed current to a biological subject includes generating a pulsed current stimulus having an amplitude current density raging from about 0.01 mA/cm² to about 10 mA/cm²; a pulse duration ranging from about 50 microseconds to about 100 milliseconds; and a pulse frequency ranging from about 0.1 Hertz to about 500 Hertz; and a duty cycle ranging from 1% to 90%.
item 21. The iontophoresis method of item 10, wherein concurrently delivering the continuous direct current having an average current density of about 0.2 mA/cm² and the pulsed current to a biological subject includes generating a pulsed alternating current stimulus having an amplitude current density of about 0.2 mA/cm2; a pulse duration of about 500 microseconds; and a pulse frequency of about 200 Hertz.
item 22. The iontophoresis method of item 10, wherein the pulsed current stimulus comprises an average current density raging from about 0.01 mA/cm² to about 10 mA/cm²; a pulse width ranging from about 50 microseconds to about 100 milliseconds; at least one pulse train having from about 2 to about 20 pulses; a pulse frequency ranging from about 0.1 Hertz to about 500 Hertz; and a duty ranging from 1% to 90%.
item 23. The iontophoresis method of item 10, wherein the pulsed current stimulus comprises an average current density raging from about 0.01 mA/cm² to about 10 mA/cm²; a pulse width ranging from about 50 microseconds to about 100 milliseconds; at least one pulse train having from about 2 to 20 pulses with alternating polarity; a pulse frequency ranging from about 0.1 Hertz to about 500 Hertz; and a duty cycle ranging from 1% to 90%.
item 24. The iontophoresis method of item 10, wherein concurrently delivering the continuous direct current and the pulsed current to a biological subject includes generation a pulsed current having sinusoidal waveforms, non-sinusoidal waveforms, or combinations thereof.
item 25. The iontophoresis method of item 10, wherein concurrently delivering the continuous direct current and the pulsed current to a biological subject includes generation a pulsed current having periodic square waveforms, rectangular waveforms, saw tooth waveforms, spiked waveforms, trapezoidal waveforms, triangle waveforms, exponential decay, or combinations thereof.
item 26. The iontophoresis method of item 10, further comprising:
   a. transdermally delivering an ellagic acid composition including
   b. one or more of ellagic acid, an ellagic acid derivative, an ion of ellagic acid, and an ion of an ellagic acid derivative present in amounts ranging from about 0.01% to about 30% by weight; and
   c. a polar solvent present in an amount ranging from about 0.1 % by weight to about 99% by weight.
item 27. An iontophoresis composition, comprising:
   a. one or more of ellagic acid, an ellagic acid derivative, an ion of ellagic acid, and an ion of an ellagic acid derivative present in amounts ranging from about 0.01% to about 30% by weight; and
   b. a polar solvent present in an amount ranging from about 0.1 % by weight to about 99% by weight.
item 28. The iontophoresis composition of item 11, further comprising:
   a. one or more anionic, cationic, nonionic, or amphoteric polymers in an amount ranging from about 0.01 % by weight to about 10%;
   b. one or more anionic, cationic, nonionic, or amphoteric surfactants in an amount preferably ranging from about 0.01 % by weight to about 30%;
   c. one or more penetrating agents in an amount ranging from 0.1 % by weight to about 30%;
   d. one or more preservatives agents in an amount ranging from 0.01% % by weight to about 5 %;
   e. one or more buffering agents in an amount ranging from 0.01% % by weight to about 10%;
   f. one or more exfoliating agents in an amount ranging from 0.1% % by weight to about 10%.
item 29. The iontophoresis composition of item 11, further comprising:
   a. Deionized water present in an amount ranging from about 20% by weight to about 60% by weight;
   b. Propylene glycol present in an amount ranging from about 0% by weight to about 20% by weight; and
   c. Sodium Hydroxide present in an amount ranging from about 0% by weight to about 1% by weight.
item 30. The iontophoresis composition of item 13, further comprising:
   a. Acrylates/C10-30 alkyl acrylate cross polymer present in an amount ranging from about 0% by weight to about 1% by weight;
   b. Cetyl alcohol present in an amount ranging from about 0 % by weight to about 1% by weight;
   c. Diisopropyl sebacate present in an amount ranging from about 0 % by weight to about 9% by weight;
   d. Dimethicone present in an amount ranging from about 0% by weight to about 5% by weight;
   e. Ethylparaben present in an amount ranging from about 0% by weight to about 1% by weight;
   f. Fragrance present in an amount ranging from about 0 % by weight to about 1% by weight;
   g. Ginkgo Biloba extract present in an amount ranging from about 0% by weight to about 1% by weight;
   h. Glycerin present in an amount ranging from about 0% by weight to about 14% by weight;
   i. Glyceryl Stearate and polyethylene glycol-100 stearate present in an amount ranging from about 0% by weight to about 3% by weight;
   j. Glycyrrhiza Glabra root extract present in an amount ranging from about 0% by weight to about 1% by weight;
   k. Helianthus Annuus seed extract present in an amount ranging from about 0% by weight to about 1% by weight;
   l. Hydrolyzed soy flour present in an amount ranging from about 0% by weight to about 1% by weight;
   m. Mentha Piperita extract present in an amount ranging from about 0% by weight to about 1% by weight;
   n. Methyl gluceth-20 present in an amount ranging from about 0% by weight to about 6% by weight;
   o. Methylparaben present in an amount ranging from about 0% by weight to about 1% by weight;
   p. Octyldodecanol present in an amount ranging from about 0% by weight to about 4% by weight;
   q. Phenoxyethanol present in an amount ranging from about 0% by weight to about 1% by weight;
   r. Polyacrylamide and C13-14 isoparaffin and laureth-7 present in an amount ranging from about 0% by weight to about 2% by weight;
   s. Polypropylene glycol-5-ceteth-20 present in an amount ranging from about 0% by weight to about 1 % by weight;
   t. Rosa Centifolia flower extract present in an amount ranging from about 0% by weight to about 1% by weight;
   u. Salicylic acid present in an amount ranging from about 0% by weight to about 1% by weight;
   v. Stearic acid present in an amount ranging from about 0% by weight to about 2% by weight;
   w. Tetrasodium ethylenediaminetetraacetic acid present in an amount ranging from about 0% by weight to about 1% by weight;
   x. Tocopherol present in an amount ranging from about 0% by weight to about 1% by weight;
   y. Xanthan gum present in an amount ranging from about 0% by weight to about 1% by weight; and
   z. Yeast extract present in an amount ranging from about 0% by weight to about 1% by weight.
item 31. The iontophoresis composition of item 11, further comprising:
   a. a pH ranging from about 4 to about 8.

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

### DESCRIPTION OF THE DRAWINGS

FIGURE 1 is a schematic illustration of an iontophoretic system according to one embodiment.
FIGURE 2 is a plot of a waveform according to one embodiment.
FIGURE 3 is a plot of a waveform according to one embodiment.
FIGURE 4 is a plot of a waveform according to one embodiment.
FIGURE 5 is a graph comparing different pH composition embodiments for ellagic acid administration according to one embodiment.
FIGURE 6 is a graph comparing ellagic acid administration (passive diffusion versus iontophoresis) according to one embodiment.
FIGURE 7 is a graph comparing different waveform embodiments for ellagic acid administration according to one embodiment.

The foregoing aspects and many of the attendant advantages of this invention will become more readily appreciated as the same become better understood by reference to the following detailed description, when taken in conjunction with the accompanying drawings, wherein:

### DETAILED DESCRIPTION

Ellagic acid (International Union of Pure and Applied Chemistry name, 2,3,7,8-Tetrahydroxy-chromeno[5,4,3-cde]chromene-5,10-dione) has many therapeutic uses. The present disclosure includes one or more methodologies or technologies for using electrical currents to deliver novel composition of ellagic acid or ellagic acid derivatives or their ions into the skin are disclosed. In an embodiment, the skin of the subjects being administered ellagic acid includes the skin of any mammal. In an embodiment, the administration of ellagic acid is conducted on live, human beings. In an embodiment, the methods are applied to any mammal, human or otherwise.

The present disclosure also includes one or more methodologies or technologies for delivering novel composition of ellagic acid or ellagic acid derivatives or their ions into the skin using iontophoresis. Iontophoresis is the process of conducting an electrical current for transporting molecules into skin of a biological subject. In an embodiment, the technique involves the application of an electrical current to a charged molecule by using a similarly charged electrode as the molecule of interest to produce a repulsive effect that drives the charged molecules away from the electrode and into the skin. In an embodiment, the effect of simple ionization (electromigration) is the main mechanism by which iontophoresis produces its transport properties. However, there are additional mechanisms called electro-osmosis and electroporation that are produced by an electrical current. Electro-osmosis induces a flow of solvent that carries uncharged molecules in the anode-to-cathode direction. Electroporation increases the permeability of lipid bilayers such as those present in the stratum corneum and in individual skin cell membranes resulting in reversible micropores formed in the lipid bilayers, briefly increasing skin permeability at higher voltages. In an embodiment, "iontophoresis" is the technique of using an electrical current to deliver molecules into the skin regardless whether transport of the molecule is via electromigration, electro-osmosis, or electroporation.

Since many active molecules in skin care compositions have ionic forms, iontophoresis can be an effective tool for the administration of these active molecules.

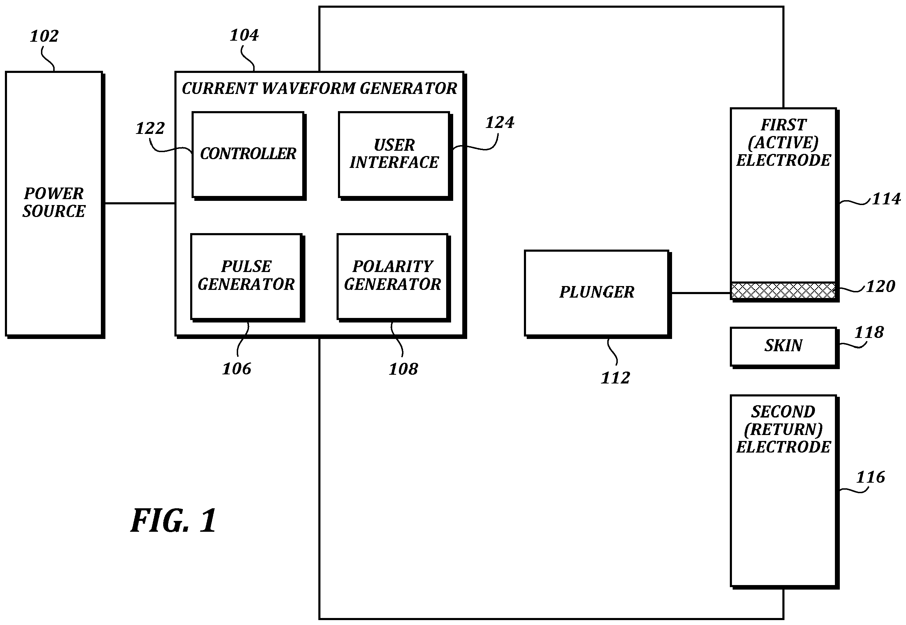

FIGURE 1 shows an iontophoresis system in which one or more methodologies or technologies can be implemented such as, for example, transporting an aqueous Ellagic acid composition into the skin of a user. In an embodiment, the iontophoresis system includes an iontophoresis device having an assembly including a power source 102, a current waveform generator 104, a first (active) electrode 114, at least one second (return) electrode 116, a plunger 112, and a reservoir 120 at the end of the first electrode. It is to be appreciated that iontophoresis devices are available with additional features or fewer features. Therefore, other devices may include many other components that are not being shown or exclude some of the components that are shown. The purpose of FIGURE 1 is to illustrate and describe some of the major functional components of an iontophoresis device used to carry out one or more of the various embodiments of the methods for the application of Ellagic acid disclosed herein. It is to be appreciated that implicit and inherent in FIGURE 1 are the many electrical components and circuits used to carry out the functions of the iontophoresis device. In an embodiment, the iontophoresis device is packaged as a hand-held device that is suitable for carrying in one hand during treatment. Treatment using hand-held devices includes constantly moving the iontophoresis device over the skin so that the active electrode 114 is moved across the surface of the skin while making contact. In an embodiment, the iontophoresis device is packaged as a stationary desktop device, and the active electrode 114 is stationary and applied to a single location on the skin, such as through an adhesive. The major functional components of the iontophoresis device will now be described.

In an embodiment, the device includes a power source 102. In an embodiment, the device includes one or more power sources 102. Non-limiting examples of power sources include one or more button cells, chemical battery cells, a fuel cell, secondary cells, lithium ion cells, micro-electric patches, nickel metal hydride cells, silver-zinc cells, capacitors, super-capacitors, thin film secondary cells, ultra-capacitors, zinc-air cells, or the like. Further non-limiting examples of power sources include one or more generators (e.g., electrical generators, thermo energy-to-electrical energy generators, mechanical-energy-to-electrical energy generators, micro-generators, nano-generators, or the like) such as, for example, thermoelectric generators, piezoelectric generators, electromechanical generators, or the like. In an embodiment, the power source includes at least one rechargeable power source. In an embodiment, the power source 102 includes one or more micro-batteries, printed micro-batteries, thin film batteries, fuel cells (e.g., biofuel cells, chemical fuel cells etc.), and the like. In an embodiment, the power source 102 includes one or more rechargeable batteries.

In an embodiment, the power source 102 provides electrical current to power the various circuits and devices. In an embodiment, a battery is used as the power source. Additionally, in an embodiment, an alternating power source coupled to a transformer can be connected to the power source 102. In an embodiment, the iontophoresis device is plugged into a wall socket. In an embodiment, the power source 102 produces direct current. The power source 102 has a negative pole and a positive pole. Generally, positive polarity will be applied to the first electrode 114. However, through circuitry and electrical devices, such as switches, the polarities of the first 114 and second 116 electrodes will be reversed momentarily to achieve the pulse current waveforms.

In an embodiment, the power source 102 is connected to the current waveform generator 104. The current waveform generator 104 functions to provide various types of current waveforms. The current generator 104 is connected to the first 114 and the second 116 electrodes and is able to apply a potential across the electrodes to supply electrical current in various waveforms described herein. Toward that end, the current waveform generator 104 includes a pulse generator 106 and a polarity switch 108. Functionally, the pulse generator 106 creates pulses of current of controlled amplitude and duration. Functionally, the polarity switch 108 controls the polarity at the first electrode 114 and second electrode 116. In an embodiment, the polarity switch 108 maintains the polarities of the first 114 and second 116 electrodes unchanged. In an embodiment, the polarity switch 108 switches OFF the first and second 116 electrodes. In an embodiment, the polarity switch 108 switches ON the first and second 116 electrodes in pulses at a predefined rate and duration. In an embodiment, the polarity switch 108 reverses the polarities of the first 114 and second 116 electrodes at a predefined rate or interval. In an embodiment, the polarity switch 108 is configured to maintain a constant polarity, generate pulses, or reverse polarity for predefined durations, sequentially or in any order.

In an embodiment, the iontophoresis device includes circuitry operably coupled to the electrode assembly and configured to concurrently generate at least a continuous direct current stimulus and a pulsed current stimulus at a first electrode. In an embodiment, the continuous direct current stimulus and the pulsed current stimulus of a character and for a duration sufficient to transdermally deliver the ellagic acid composition to a biological subject. In an embodiment, circuitry includes, among other things, one or more computing devices such as a processor (e.g., a microprocessor), a central processing unit (CPU), a digital signal processor (DSP), an application-specific integrated circuit (ASIC), a field-programmable gate array (FPGA), or the like, or any combinations thereof, and can include discrete digital or analog circuit elements or electronics, or combinations thereof. In some embodiments, circuitry includes one or more ASICs having a plurality of predefined logic components. In some embodiments, circuitry includes one or more FPGA having a plurality of programmable logic components.

In an embodiment, the device is configured to electrochemically deliver an ellagic acid through the skin by applying a DC, unidirectional current. In an embodiment, the device includes circuitry configured to apply a unidirectional direct current of a character and for a duration sufficient to deliver an ellagic acid through the skin.

In an embodiment, the iontophoresis device includes a controller 122. Functionally, the controller 122 will receive input from the user interface 124 and, in conjunction with the pulse generator 106 and the polarity switch 108, control the current density waveforms at the specifications input by the user/operator. In an embodiment, the controller 122, pulse generator 106, and polarity switch 108 are implemented in hardware components, such as analog circuitry, digital circuitry, microprocessors, or combinations thereof, or software components.

In an embodiment, the controller 122 has instructions for guiding a user to input the various parameters depending on the waveform treatment that is to be applied. The user interface 124 can prompt the user for the information. In an embodiment, the controller 122 will request the user to select the output from a constant direct current, a pulse wave, or both a constant and a pulse wave for the current density output waveform. Once the controller 122 receives input of the one or more wave types, the controller 122 prompts the user on the user interface 124 for parameters corresponding to the selected wave output type or types.

In an embodiment, the iontophoresis device includes a user interface 124. In an embodiment, the user interface 124 is used for data entry, data communication, control, information display, etc. In an embodiment, the user interface 124 includes an alphanumeric keyboard and display. In an embodiment, the user interface 124 includes directional arrow buttons and an enter button. In an embodiment, the alphanumeric keyboard is implemented as a touch screen display. In an embodiment, the input of wave parameters is through the use of text boxes. In an embodiment, the input of wave parameters is through the use of scrolling menus.

In an embodiment, the user interface 124 is configured to communicate a variety of prompts for the user to input information. In an embodiment, the user interface 124 prompts the use for duration of the treatment step. In an embodiment, the duration of the treatment step is the sum of time of the application of an electrical current of any one or more wave types and includes the time when electrical current is off for pulse waves. For example, a pulse wave set with a duty cycle of 50% has the electrical current off during 50% of the time, meaning the each pulse is one-half of each pulse cycle. However, the treatment duration will include the off period of the pulse cycle. In an embodiment, the user interface 124 provides options to allow the user to input whether the current density output will be constant direct current, pulse, alternating pulse, or any combination, and the duration of each wave type. In an embodiment, the user interface 124 prompts the user whether different wave types are superimposed on each other. For example, a pulse wave can be superimposed on a direct current wave.

In an embodiment, the user interface 124 prompts the user whether different wave types are combined in sequence. In an embodiment, the user interface 124 prompts the user to input the current density values to output to each wave type. In an embodiment, the user interface 124 prompts the user to input the polarity at the first electrode 114, including positive, negative, or both with the corresponding constant or pulsed current output. In an embodiment, the user interface 124 prompts the user to input the electrodes' cross-sectional areas. In an embodiment, the user interface 124 prompts the user to input skin temperature. In an embodiment, the user interface 124 prompts the user to input the frequency of pulses, the maximum and minimum amplitudes of pulses, and the duration of pulses. In an embodiment, the user interface 124 prompts the user to input the % duty cycle of unidirectional pulses. In an embodiment, the user interface 124 prompts the user to input the percentage (%) of respective biphasic pulses. In an embodiment, the user interface 124 prompts the user to specify the time between pulses.

In an embodiment, the user interface 124 prompts the user to input the pulse wave shape, including triangular, square, sinusoidal, or sawtooth. In an embodiment, the user interface 124 prompts the user to input the treatment area. In an embodiment, the user interface 124 prompts the user to input the duration of the treatment. In an embodiment, the user interface 124 prompts the user to specify whether to apply alternating negative and positive pulses. In an embodiment, the user interface 124 prompts the user to specify whether pulses are to be monophasic or biphasic. A monophasic pulse means that pulsed electrical current travels in one direction. A biphasic pulse means that pulsed electrical current travels in two directions or reverses directions. In an embodiment, the user interface 124 prompts the user to specify a ratio of negative to positive pulses or a ratio of positive to negative pulses. In an embodiment, the user interface 124 prompts the user to combine any direct current constant waveform with any pulse waveform to provide two or more different waveforms in a single treatment. In an embodiment, the user interface 124 prompts the user to apply two or more waveforms concurrently or alternately. In an embodiment, the user interface 124 prompts the user to input the duration of each waveform and the cycle time of each waveform if the waveforms alternate. In an embodiment, the user interface 124 prompts the user for a pulse interval duration.

In an embodiment, when using two or more waveforms in a single treatment, the user interface 124 prompts the user to specify the treatment durations of the different waveforms, and how often each waveform cycles. In an embodiment, when direct current is used in combination with pulses, the user interface 124 prompts the user to specify the time interval between pulses.

In an embodiment, the controller 122 carries out logic routines to direct the user interface 124 to present to the user the appropriate prompts so that the wave type information is entered. The controller 122 then uses the wave type parameters to generate, via the pulse generator 106 and the polarity switch 108, the appropriate wave according to the user entered parameters.

In an embodiment, the first electrode 114, also referred to as the active electrode, is connected to the power source 102 through the waveform generator 104. The first electrode 114 includes a reservoir 102 on the end thereof to hold an ellagic acid composition. In an embodiment, the reservoir 120 is a hollow indentation on the end of the first electrode 114, wherein the reservoir is used to contain a gel or gel-like ellagic acid composition. Alternatively, in an embodiment, the reservoir 120 is an absorbent material to contain the ellagic acid composition. Generally, the design considerations of the first electrode 114 contemplates the first electrode 114 having positive polarity. When the iontophoresis device is packaged as a hand-held device, the first electrode 114 may be provided with a type of roll-on applicator, such as a ball and socket fed by the plunger 112.

The skin 118 represents the load on the system.

In an embodiment, the second electrode 116, also referred to as the return electrode, is connected to the power source 102 through the waveform generator 104. The polarity of the second electrode 116 is maintained by the waveform generator 104 to be the opposite of the polarity of the first electrode 114. Generally, the design considerations of the second electrode 116 contemplates the second electrode 116 having negative polarity. In an embodiment, the second electrode 116 is a hand-held electrode that is held by the person receiving the treatment. In other embodiments, the second electrode 116 has an insulated cover and an exposed tip so that the second electrode may be held by the device user and applied on the skin of the person receiving the treatment.

In an embodiment, the reservoir 120 on the first electrode 114 is connected to a plunger 112. Functionally, the plunger 112 replenishes the ellagic composition in the reservoir 120. In an embodiment, the plunger 112 includes a piston and push pod within a cylindrical container. In an embodiment, the plunger 112 can be operated by a trigger mechanism that is operated during treatment by the user of the iontophoresis device.

In an embodiment, an iontophoresis assembly includes active (donor) and at least one return (counter) electrode assemblies, a skin contacting layer, and an active agent layer. In an embodiment, an iontophoresis assembly includes active and at least one return electrode assemblies having a multi-laminate construction. In an embodiment, an iontophoresis assembly includes a multi-laminate construction configured to deliver an active agent composition through passive diffusion or iontophoresis.

In an embodiment, an iontophoresis assembly includes active and at least one return electrode assemblies, each formed by multiple layers of polymeric matrices. In an embodiment, an iontophoresis assembly includes electrode assemblies having a conductive resin film electrode layer, a hydrophilic gel reservoir layer, an aluminum foil conductor layer, and an insulating backing layer.

In an embodiment, an iontophoresis assembly comprises an iontophoresis patch design. In an embodiment, an iontophoresis assembly comprises an iontophoresis multi-laminate design. In an embodiment, an iontophoresis assembly comprises an iontophoresis face mask design. In an embodiment, an iontophoresis assembly comprises an iontophoresis flexible substrate design.

In an embodiment, an iontophoresis assembly includes one or more active agents stored in a reservoir such as a cavity, a gel, a laminate, a membrane, a porous structure, a matrix, a substrate, or the like. Non-limiting examples of active agents include electrically neutral agents, molecules, or compounds capable of being delivered via electro-osmotic flow. In an embodiment, neutral agents are carried by the flow of, for example, a solvent during electrophoresis. In an embodiment, an iontophoresis assembly includes an electrode and a reservoir containing an effective amount of an ellagic acid composition.

In an embodiment, a reservoir includes any form or matter employed to retain an element, a composition, a compound, active agent, a pharmaceutical composition, and the like, in a liquid state, solid state, gaseous state, mixed state or transitional state. In an embodiment, a reservoir includes one or more ion exchange membranes, semi-permeable membranes, porous membranes or gels capable of at least temporarily retaining an element, a composition, a compound, active agent, a pharmaceutical composition, electrolyte solution, and the like.

In an embodiment, a reservoir includes one or more cavities formed by a structure. In an embodiment, a reservoir serves to retain an element, a composition, a compound, active agent, a pharmaceutical composition, electrolyte solution, and the like prior to the discharge of such by electromotive force or current into a biological interface. In an embodiment, an iontophoresis assembly includes one or more an ion exchange membrane may be positioned to serve as a polarity selective barrier between the active agent reservoir and a biological interface.

In an embodiment, an iontophoresis assembly includes an electrode and a reservoir containing an effective amount of an ellagic acid composition.

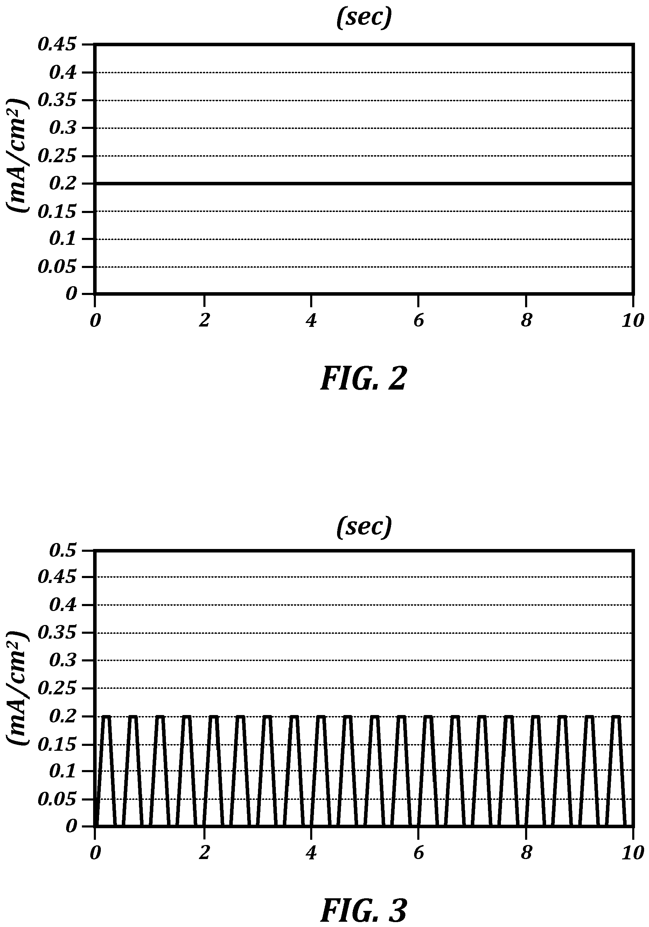

FIGURES 2-4 illustrate embodiments of representative current density waveforms output by the iontophoresis device and assembly to administer ellagic acid into the skin.

Referring to FIGURE 2, a first current density waveform is illustrated for the administration of ellagic acid into the skin via the use of iontophoresis. The current density waveform is controlled about a constant value for the duration or any part of the iontophoresis treatment. A current density waveform controlled at a constant value is referred to as direct current. In an embodiment, and the terms "constant current," galvanic current and "direct current" are interchangeable. In an embodiment, a negative current density means that the polarity at the first electrode 114 is negative. The second electrode 116 has positive polarity when the first electrode has negative polarity. Following convention, this means that current flows from the second positive electrode 116 to first negative electrode 114. Conversely, when the first electrode 114 has positive polarity and the second electrode 116 has negative polarity, current flows from the first positive electrode 114 to the second negative electrode 116, and this will be represented on a graph by a positive value of current density. Conventionally, electrons will be defined to flow in the opposite direction to current, i.e., from negative polarity to positive polarity. Current density is defined as ampere units per area units (of the cross section of the active electrode).

While FIGURE 2 depicts a certain direct current density value and duration, it should be appreciated that the illustrated values are exemplary. In an embodiment of the direct current waveform of FIGURE 2, the current density is constant and controlled at or below 0.5 mA/cm². In an embodiment of the direct current waveform of FIGURE 2, the current density is constant and controlled at or below 0.2 mA/cm². In an embodiment of the direct current waveform of FIGURE 2, the current density is constant and controlled between 0.01 mA/cm² to 0.5 mA/cm². In an embodiment of the direct current waveform of FIGURE 2, the current density is constant and controlled at any one of the following values, 0.01, 0.05, 0.1, 0.15, 0.2, 0.25, 0.3, 0.35, 0.4, 0.45, 0.5 mA/cm² or in a range between any two values serving as endpoints. In an embodiment of the direct current waveform of FIGURE 2, the amplitude is constant at any of the above values and electrical current is applied for a duration of at least 1 minute. In an embodiment of the direct current waveform of FIGURE 2, the amplitude is constant at any of the above values and electrical current is applied for a duration of from 10 to 20 minutes. In an embodiment of the direct current waveform of FIGURE 2, the amplitude is constant at any of the above values and electrical current is applied for a duration (in minutes) of 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, or any range between any two values serving as endpoints or any range between any two values serving as endpoints. In the embodiments of the direct current waveform of FIGURE 2, the first electrode 114 is positive and the second electrode 116 is negative for duration of the waveform.

In an embodiment of FIGURE 2, a method for transdermal administration of ellagic acid and/or ellagic acid derivatives comprises iontophoretically delivering an ellagic acid composition having from about 0.01% by weight to about 30% by weight of ellagic acid or ellagic acid derivatives or their ions. In an embodiment of FIGURE 2, iontophoretically delivering the ellagic acid composition includes applying a potential to a device having a first electrode in contact with a first skin surface where ellagic acid or ellagic acid derivatives or both are to be administered and a second electrode in contact with a second skin surface, and wherein the first electrode is connected to a reservoir containing the ellagic acid composition; and conducting electrical current between the first and second electrodes, wherein the current passes through the ellagic acid composition, and a polarity and magnitude of current density at the first electrode is sufficient to initiate transdermal transport of ellagic acid ions or ellagic acid derivative ions or both. In an embodiment of FIGURE 2, iontophoretically delivering the ellagic acid composition includes causing an iontophoresis assembly to generate a positive polarity at a first electrode and controlling a current density of at most 0.5 mA/cm² for at least 1 minute. In an embodiment of FIGURE 2, iontophoretically delivering the ellagic acid composition includes applying a current density of from 0.05 mA/cm² to 0.5 mA/cm². In an embodiment of FIGURE 2, iontophoretically delivering the ellagic acid composition includes applying a current density from 1 to 30 minutes. In an embodiment of FIGURE 2, the ellagic acid composition has a pH from 4.0 to 8. In an embodiment of FIGURE 2, the transdermal transport of ellagic acid ions or ellagic acid derivative ions includes electromigration or electro-osmosis or both of the ellagic acid ions or the ellagic acid derivative ions.

Referring to FIGURE 3, a second current density waveform is illustrated for the administration of ellagic acid into the skin via the use of iontophoresis. The current density waveform is controlled in positive pulses (polarity is positive at electrode 114) for the duration or any part of the iontophoresis treatment. The pulses of FIGURE 3 are monophasic, meaning that current travels in one direction. A pulse of FIGURE 3 has a maximum amplitude. The pulse waveform will increase from a minimum amplitude, reach the maximum amplitude, and then decrease to the minimum amplitude, reside at the minimum amplitude, and the cycle will repeat. A pulse is counted starting from the minimum amplitude, reaching the maximum amplitude, and then returning to the minimum amplitude. Thus, a pulse does not include the period of the minimum amplitude. A pulse cycle does include the period at the minimum amplitude. When the frequency of pulse cycles is stated, the durations of the maximum and minimum amplitudes are not changed.

In an embodiment, the pulse wave is expressed to have a % duty cycle. In an embodiment, expressing a % duty cycle with respect to a pulse wave means that the electrical current is on for the % duty cycle. For example, 50 % duty cycle means electrical current is on for 50% and off for 50% of the pulse cycle, 30% duty cycle means electrical current is on for 30% and off for 70% of the pulse cycle. In an embodiment, the % duty cycle of unidirectional pulses is 5, 10, 15, 20, 25, 30, 35, 40, 45, 50, 55, 60, 65, 70, 75, 80, 85, 90, 95, or any range between any two values serving as endpoints. In an embodiment, the % of a respective biphasic pulse is 5, 10, 15, 20, 25, 30, 35, 40, 45, 50, 55, 60, 65, 70, 75, 80, 85, 90, 95, or any range between any two values serving as endpoints. In an embodiment, the pulse, meaning the "on" period can be expressed as a duration having units of time. In an embodiment, the pulse "off" period can be expressed as a duration. In an embodiment, the frequency of a pulse will be expressed in hertz, meaning cycles per second. In an embodiment, the pulses can be reversed by alternating the polarities of the first and second electrodes between negative and positive. In an embodiment, biphasic pulses, alternating pulses, bidirectional pulses, and reverse pulses mean the same. In an embodiment, negative current density pulses will be followed by positive current density pulses, without residing at a minimum. A pulse waveform including both negative and positive current density pulses will include a maximum value for negative pulses, a maximum value for positive pulses, and the values do not have to be the same. Further, in an embodiment, the duration of negative current density pulse does not have to be the same duration of a positive current density pulse. In an embodiment, the duration of pulses does not have to be the same duration, regardless whether the pulses are negative or positive.

In an embodiment, a pulse waveform can combine two or more pulse waveforms. In an embodiment, a pulse waveform can include negative pulses, followed by positive pulses. Thus, having a maximum and minimum amplitude for the negative pulses and a maximum and a minimum amplitude for the positive pulses.

While FIGURE 3 depicts certain current density values of the maximum and minimum pulse amplitudes and pulse duration, it should be appreciated that the illustrated values are exemplary only. In an embodiment of the current waveform of FIGURE 3, the current density is controlled in pulses and each pulse maximum is controlled at most at 0.2 mA/cm² and the minimum amplitude is 0. In an embodiment of the current waveform of FIGURE 3, the current density is controlled in pulses and each pulse maximum is controlled at or below 1 mA/cm² and the minimum amplitude is 0. In an embodiment of the current waveform of FIGURE 3, the current density is controlled in pulses and each pulse maximum is controlled between 0.2 mA/cm² to 1 mA/cm² and the minimum amplitude is 0. In an embodiment of the current waveform of FIGURE 3, the current density is controlled in pulses and each pulse maximum is controlled at or below 0.2 mA/cm² and the minimum amplitude is 0. In an embodiment of the current waveform of FIGURE 3, the current density is controlled in pulses and each pulse maximum is controlled at 0.01, 0.05, 0.1, 0.15, 0.2, 0.25, 0.3, 0.35,0.4, 0.45, 0.5 ... mA/cm² or in the range between any two values serving as endpoints.

In an embodiment, the pulse has a positive constant slope (other than vertical) to the maximum amplitude, followed by a duration at the constant maximum amplitude, followed by a negative constant slope (other than vertical) to 0, followed by a duration at 0. In an embodiment, the minimum can be other than 0. In an embodiment, the slope can be other than constant, such as exponential. In an embodiment of the current waveform of FIGURE 3, the pulses are not triangular. In an embodiment of FIGURE 3, the pulses are a square wave, wherein the maximum and the minimum amplitudes are of the same duration. In an embodiment of the current waveform of FIGURE 3, the pulses are not square wave. Some embodiments of non-square waves include sinusoidal, sawtooth, and triangular pulse waves, rectangular pulse, exponential decay, and the like or combinations thereof.

In an embodiment of FIGURE 3, the duration of the maximum amplitude of the pulses is less than the duration of the minimum amplitude between pulses. In an embodiment of FIGURE 3, the duration of the maximum amplitude of the pulses is greater than the duration of the minimum amplitude between pulses. In an embodiment of the current waveform of FIGURE 3, the minimum amplitude is 0 mA/cm² (milliampere per centimeter squared). In an embodiment of FIGURE 3, the minimum amplitude is greater than 0 mA/cm² (meaning positive with respect to FIGURE 3). In an embodiment of the current waveform of FIGURE 3, the maximum (and minimum) amplitude can increase from pulse to pulse. In an embodiment of the current waveform of FIGURE 3, the maximum (and minimum) amplitude can decrease from pulse to pulse. In an embodiment of the current waveform of FIGURE 3, the maximum (and minimum) amplitude can increase from pulse to pulse, and then decrease from pulse to pulse, and repeat. In an embodiment of the current waveform of FIGURE 3, the current density is controlled in pulses at any of the above values and the rate of pulses is from 1 hertz to 2000 hertz. In an embodiment of the current waveform of FIGURE 3, the current density is controlled in pulses at any of the above values and each pulse cycle has a duration of between 5 microseconds to 500 milliseconds. In an embodiment of the current waveform of FIGURE 3, the current density is controlled in pulses, the rate of pulses is 2000 Hz, and each pulse has a duration of 250 microseconds.

In an embodiment of the current waveform of FIGURE 3, the iontophoresis treatment is applied for a duration of from 1 minute to 5 minutes. In an embodiment of the current waveform of FIGURE 3, the iontophoresis treatment is applied for a duration (in minutes) of 1, 1.5, 2, 2.5, 3, 3.5, 4, 4.5, 5 or any range between any two values as endpoints. In an embodiment of the pulse waveform of FIGURE 3, the electrical current is applied in pulses for a duration (in minutes) of 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, or any range between any two values serving as endpoints, or any range between any two values serving as endpoints. In the embodiments of the current waveform of FIGURE 3, the first electrode 114 has positive polarity and the second electrode 116 has negative polarity when the pulses extend above 0. However, the first electrode 114 is negative and the second electrode 116 is positive when the pulses extend above 0. Thus, indicating a reversal in the direction of current.

In an embodiment of FIGURE 3, a method for transdermal administration of ellagic acid and/or ellagic acid derivatives comprises iontophoretically delivering an ellagic acid composition having from about 0.01% by weight to about 30% by weight of ellagic acid or ellagic acid derivatives or their ions. In an embodiment of FIGURE 3, iontophoretically delivering the ellagic acid composition includes applying a potential to a device having a first electrode in contact with a first skin surface where ellagic acid or ellagic acid derivatives or both are to be administered and a second electrode in contact with a second skin surface, and wherein the first electrode is connected to a reservoir containing the ellagic acid composition; and conducting electrical current between the first and second electrodes, wherein the current passes through the ellagic acid composition, and a polarity and magnitude of current density at the first electrode is sufficient to initiate transdermal transport of ellagic acid ions or ellagic acid derivative ions or both. In an embodiment of FIGURE 3, iontophoretically delivering the ellagic acid composition includes generating positive polarity at a first electrode and controlling current density in pulses of at most 1 mA/cm² for at least 1 minute. In an embodiment of FIGURE 3, the method further comprises controlling current density in pulses of 0.05 mA/cm² to 1 mA/cm². In an embodiment of FIGURE 3, the method further comprises controlling current density in pulses for 1 to 30 minutes. In an embodiment of FIGURE 3, the pulses are applied at a rate of at most 2500 hertz. In an embodiment of FIGURE 3, the pulses are applied at a rate of 2000 hertz, wherein each pulse is 0.00025 seconds. In an embodiment of FIGURE 3, the transdermal transport of ellagic acid ions or ellagic acid derivative ions includes electromigration or electro-osmosis or both of the ellagic acid ions or the ellagic acid derivative ions.

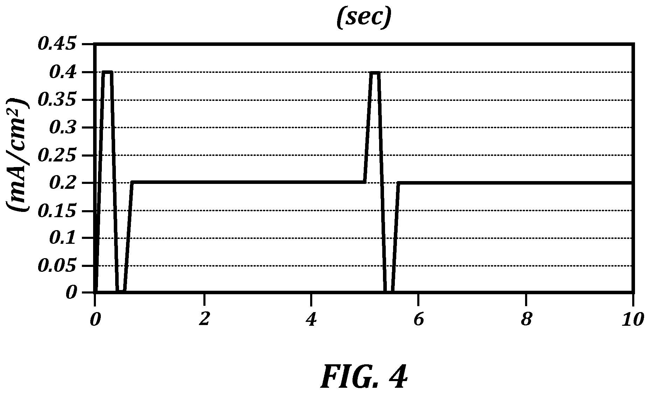

Referring to FIGURE 4, a third current density waveform is illustrated for the administration of ellagic acid into the skin via the use of iontophoresis. In an embodiment, the current density waveform is controlled in positive monophasic pulses or biphasic pulse and between pulses. In an embodiment, the current density is controlled as constant direct current. FIGURE 4 represents a synchronous mode of applying two waveforms. The two waveforms in combination are applied synchronously for the duration or any part of the iontophoresis treatment. Specifically, the pulse wave has an on and off period. The superposition of the pulse wave on top of constant direct current causes the current profile to show the pulse beginning at the constant value of the direct current constant current. The pulse reaches a maximum for the predetermined duration and then the profile goes to 0. After the off period from the 0 value, the constant direct current is applied until then next pulse. Thus, the current density of the waveform can be described as the addition of constant direct current of a first amplitude with a pulse of a second amplitude, wherein the pulse has an off period before applying the direct current again. A pulse is counted starting from the direct current amplitude, reaching the maximum pulse amplitude, and then returning to the minimum amplitude or 0. Thus, a pulse does not include the period of the minimum amplitude at 0. A pulse cycle does include the period at the minimum amplitude. When the frequency of pulse cycles is stated, the durations of the maximum and minimum amplitudes are the same.

In an embodiment, the pulse is expressed to have a % duty cycle. In an embodiment, expressing a % duty cycle with respect to a pulse wave means that the electrical current is on for the % duty cycle. For example, 50 % duty cycle means electrical current is on for 50% and off for 50% of the pulse cycle, 30% duty cycle means electrical current is on for 30% and off for 70% of the pulse cycle. In an embodiment, the % duty cycle of unidirectional pulses is 5, 10, 15, 20, 25, 30, 35, 40, 45, 50, 55, 60, 65, 70, 75, 80, 85, 90, 95, or any range between any two values serving as endpoints. In an embodiment, the % of a respective biphasic pulse is 5, 10, 15, 20, 25, 30, 35, 40, 45, 50, 55, 60, 65, 70, 75, 80, 85, 90, 95, or any range between any two values as endpoints. In an embodiment, the pulse, meaning the "on" period can be expressed as a duration. In an embodiment, the pulse "off" period can be expressed as a duration. In an embodiment, the pulse wave of will be expressed in hertz, meaning cycles per second. In an embodiment, the pulses can be reversed by alternating the polarities of the first and second electrodes between negative and positive. In an embodiment, biphasic pulses, alternating pulses, and reverse pulses mean the same. In an embodiment, negative current density pulses will be followed by positive current density pulses, without residing at a minimum. A pulse waveform including both negative and positive current density pulses will include a maximum value for negative pulses, a maximum value for positive pulses, and the values do not have to be the same. Further, in an embodiment, the duration of negative current density pulse does not have to be the same duration of a positive current density pulse. In an embodiment, the duration of pulses does not have to be the same duration, regardless whether the pulses are negative or positive.

In an embodiment, a pulse waveform can combine two or more pulse waveforms. In an embodiment, a pulse waveform can include negative pulses, followed by positive pulses. Thus, having a maximum and minimum amplitude for the negative pulses and a maximum and a minimum amplitude for the positive pulses.
While FIGURE 4 depicts certain current density values of the maximum and minimum pulse amplitudes and pulse duration, it should be appreciated that the illustrated values are exemplary only. In an embodiment of the current waveform of FIGURE 4, the current density is controlled in pulses and each pulse maximum is controlled at most at 0.2 mA/cm² and the minimum amplitude is 0. This means that the addition of the pulse to the direct current totals 0.4 mA/cm². In an embodiment of the current waveform of FIGURE 4, the current density is controlled as direct current in combination with pulses and each pulse maximum is at or below 0.5 mA/cm² and the minimum amplitude is 0, and direct current is controlled at or below 0.5 mA/ cm². In an embodiment of the current waveform of FIGURE 4, the current density is controlled as direct current in combination with pulses and each pulse maximum is controlled between 0.2 mA/cm² to 0.5 mA/cm² and the minimum amplitude is 0, and the direct current is controlled between 0.2 mA/cm² to 0.5 mA/cm². In an embodiment of the current waveform of FIGURE 4, the current density is controlled as direct current in combination with pulses and each pulse maximum is controlled at or below 0.2 mA/cm² and the minimum amplitude is 0, and the direct current is controlled at or below 0.2 mA/cm². In an embodiment of the current waveform of FIGURE 4, the current density is controlled as direct current in combination with pulses and the direct current and each pulse maximum is controlled at 0.01, 0.05, 0.1, 0.15, 0.2, 0.25, 0.3, 0.35,0.4, 0.45, 0.5 mA/cm² or in the range between any two values serving as endpoints.

In an embodiment of FIGURE 4, the pulses are triangular with a defined maximum and minimum, wherein the maximum and the minimum amplitudes are of the same duration. Specifically, the pulse has a positive constant slope (other than vertical) to the maximum amplitude, followed by a period at the constant maximum amplitude, followed by a negative constant slope (other than vertical) to 0, followed by a period at 0. In an embodiment, the minimum can be other than 0. In an embodiment, the slope can be other than constant, such as exponential. In an embodiment of the current waveform of FIGURE 4, the pulses are not triangular. Some embodiments of pulse wave shapes include sinusoidal, sawtooth, and square pulse waves, exponential decay, and the like or combinations thereof.

In an embodiment of FIGURE 4, the duration of the maximum amplitude of the pulses is less than the duration of the minimum amplitude between pulses. In an embodiment of FIGURE 4, the duration of the maximum amplitude of the pulses is greater than the duration of the minimum amplitude between pulses. In an embodiment of the current waveform of FIGURE 4, the minimum amplitude is 0 mA/cm². In an embodiment of FIGURE 4, the minimum amplitude is greater than 0 mA/cm² (meaning positive with respect to FIGURE 4) or negative compare to continuous current (meaning opposite polar to continuous current). In an embodiment of the current waveform of FIGURE 4, the maximum (and minimum) amplitude can increase from pulse to pulse. In an embodiment of the current waveform of FIGURE 4, the maximum (and minimum) amplitude can decrease from pulse to pulse. In an embodiment of the current waveform of FIGURE 4, the maximum (and minimum) amplitude can increase from pulse to pulse, and then decrease from pulse to pulse, and repeat.

In an embodiment of the current waveform of FIGURE 4, the current density is controlled as direct current in combination with pulses at any of the above values and the rate of pulses is from 0.2 hertz to 500 hertz, with a corresponding pulse duration of from 0.5 seconds to 0.0025 seconds. In an embodiment of the current waveform of FIGURE 4, the pulse interval is 0.002...1.0, 1.5, 2, 2.5, 3.0, 3.5, 4.0, 4.5, or 5.0 seconds. In an embodiment of the current waveform of FIGURE 4, the iontophoresis treatment is applied for a duration of from 1 minute to 5 minutes.

In an embodiment of the current waveform of FIGURE 4, the iontophoresis treatment is applied for a duration (in minutes) of 1, 1.5, 2, 2.5, 3, 3.5, 4, 4.5, 5 or any range between any two values serving as endpoints. In an embodiment of the waveform of FIGURE 4, the electrical current is applied as direct current and in pulses for a duration (in minutes) of 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, or any range between any two values serving as endpoints or any range between any two values serving as endpoints. In the embodiments, of the current waveform of FIGURE 4, the pulses are monophasic pulses. In the embodiments of the current waveform of FIGURE 4, the first electrode 114 has positive polarity and the second electrode 116 has negative polarity when the pulses extend above zero. However, the first electrode 114 is negative and the second electrode 116 is positive when the pulses extend below zero. Thus, indicating a reversal in the direction of current.

In an embodiment of FIGURE 4, a method for transdermal administration of ellagic acid and/or ellagic acid derivatives comprises iontophoretically delivering an ellagic acid composition having from about 0.01% by weight to about 30% by weight of ellagic acid or ellagic acid derivatives or their ions. In an embodiment of FIGURE 4, iontophoretically delivering the ellagic acid composition includes applying a potential to a device having a first electrode in contact with a first skin surface where ellagic acid or ellagic acid derivatives or both are to be administered and a second electrode in contact with a second skin surface, and wherein the first electrode is connected to a reservoir containing the ellagic acid composition; and conducting electrical current between the first and second electrodes, wherein the current passes through the ellagic acid composition, and a polarity and magnitude of current density at the first electrode is sufficient to initiate transdermal transport of ellagic acid ions or ellagic acid derivative ions or both. In an embodiment of FIGURE 4, iontophoretically delivering the ellagic acid composition includes generating applying a positive polarity at a first electrode, and controlling the electrical current as direct current and in pulses of at most 0.5 mA/cm² r.m.s. for at least 1 minute. In an embodiment of FIGURE 4, controlling the electrical current includes controlling the electrical current as direct current and in pulses from 0.05 mA/cm² r.m.s. to 0.5 mA/cm² r.m.s. In an embodiment of FIGURE 4, controlling the electrical current includes controlling the electrical current as direct current and in pulses from 1 to 30 minutes. In an embodiment of FIGURE 4, the pulses are applied at a rated from 1 hertz to 200 hertz, wherein each pulse is 1 second to 0.005 seconds, respectively. In an embodiment of FIGURE 4, the pulses are applied at a rate of 200 hertz, wherein each pulse is 0.005 seconds. In an embodiment of FIGURE 4, the transdermal transport of ellagic acid ions or ellagic acid derivative ions includes electromigration or electro-osmosis or both of the ellagic acid ions or the ellagic acid derivative ions.

In an embodiment, the current waveforms of FIGURES 2, 3, and 4 can be combined to provide combinations of waveforms for the administration of ellagic acid into the skin via the use of iontophoresis. In an embodiment, the current waveform of any FIGURE 2-4 is applied for a first duration, followed by a different current waveform of any FIGURE 2-4 for a second duration or vice versa. In an embodiment, two or more different current waveforms can be cycled for the entire electrical current treatment duration. The particulars of the waveform as described above for FIGURES 2-4 are similarly applicable to combined treatments applying the two or more different waveforms. That is, any one or more of the embodiments of the direct current waveform can be combined with any one or more of the pulse waveforms in sequence or concurrently, or with intervals of OFF period.

In the case of the waveforms of FIGURES 2, 3, and 4, the peak voltage at maximum peak is 99 volts. In an embodiment, the maximum duration of conducting electrical current is 120 minutes during the iontophoresis treatment.

Every embodiment of the current density waveforms described in connection with FIGURES 2, 3, and 4 and the combination of waveforms can be used with every embodiment of the ellagic acid composition disclosed herein.

In an embodiment, a composition formulated for use in iontophoresis, comprises from 0.01% by weight to 30.0% by weight ellagic acid or ellagic acid derivatives or their ions; and from 0.1 % by weight to 99% by weight of organic polar solvents, including water, wherein the composition is formulated for use in iontophoresis. In an embodiment of the composition formulated for use in iontophoresis, the organic polar solvents comprise at most 98 % by weight of the composition. In an embodiment of the composition formulated for use in iontophoresis, the ellagic acid and ellagic acid derivatives and their ions comprise at most 30% by weight of the composition. In an embodiment of the composition formulated for use in iontophoresis, the ellagic acid and ellagic acid derivatives and their ions comprise from 0.05 % by weight to 0.5 % by weight of the composition. In an embodiment of the composition formulated for use in iontophoresis, a pH of the composition is from 4.0 to 8. In an embodiment of the composition formulated for use in iontophoresis, a pH of the composition is at least 4.0. In an embodiment, the composition formulated for use in iontophoresis comprises a pH of at most 8.

In an embodiment, a composition formulated for use in iontophoresis, includes, from 0.1% by weight to 30.0% by weight ellagic acid or ellagic acid derivatives or their ions; from 1 % by weight to 99% by weight of organic polar solvents, including water.

In an embodiment, a composition formulated for use in iontophoresis, includes, from 0.1 % by weight to 30.0% by weight ellagic acid or ellagic acid derivatives or their ions; from 1 % by weight to 99% by weight of organic polar solvents, including water, wherein the organic polar solvents comprise at most 98 % by weight of the composition.

In an embodiment, a composition formulated for use in iontophoresis, includes, from 0.1% by weight to 30% by weight ellagic acid or ellagic acid derivatives or their ions; from 1 % by weight to 95% by weight of organic polar solvents, including water, wherein the ellagic acid and ellagic acid derivatives and their ions comprise at most 30% by weight of the composition.

In an embodiment, a composition formulated for use in iontophoresis, includes, from 0.1% by weight to 30% by weight ellagic acid or ellagic acid derivatives or their ions; from 1 % by weight to 99% by weight of organic polar solvents, including water, wherein the ellagic acid and ellagic acid derivatives and their ions comprise at about 0.5 %.

In an embodiment, a composition formulated for use in iontophoresis, includes, from 0.1% by weight to 30% by weight ellagic acid or ellagic acid derivatives or their ions; from 1 % by weight to 95% by weight of organic polar solvents, including water, wherein the pH of the composition is from 4.0 to 8.

In an embodiment, a composition formulated for use in iontophoresis, includes, from 0.1 % by weight to 30.0% by weight ellagic acid or ellagic acid derivatives or their ions; from 1 % by weight to 99% by weight of organic polar solvents, including water, wherein the pH of the composition is at least 4.0.

In an embodiment, a composition formulated for use in iontophoresis, includes, from 0.1% by weight to 30% by weight ellagic acid or ellagic acid derivatives or their ions; from 1 % by weight to 99% by weight of organic polar solvents, including water, wherein the pH of the composition is at most 8.

In an embodiment of the ellagic acid composition, the ellagic acid composition will include one or more of a solvent, a humectant, skin protectant, a preservative, a skin conditioner, a chelating agent, an exfoliant, an emulsifier, a cleansing agent, an emulsion stabilizer, an emollient, an antioxidant, a fragrance, a binder, viscosity increaser, a denaturant, a pH adjuster, a buffering agent, plant extracts, algae extracts, and seed extracts.

In an embodiment, the ellagic acid composition includes one or more solvents selected from water and deionized water.

In an embodiment, the ellagic acid composition includes one or more humectants. Non-limiting examples of humectants include glycerin, propylene glycol, methyl gluceth-20, Glycyrrhiza Glabra root extract, and caprylyl glycol.

In an embodiment, the ellagic acid composition includes one or more skin protectants selected from glycerin and Palmaria Palmata extract.

In an embodiment, the ellagic acid composition includes one or more preservatives selected from phenoxyethanol, methylparaben, and ethylparaben.

In an embodiment, the ellagic acid composition includes one or more skin conditioners of propylene glycol, salicylic acid, methyl gluceth-20, tocopherol, dimethicone, Rosa Centifolia flower extract, hydrolyzed soy flour, yeast extract, Helianthus Annuus seed extract, ellagic acid, caprylyl glycol, Chlorella Vulgaris extract, and Mentha Piperita extract.

In an embodiment, the ellagic acid composition includes one or more chelating agents selected from tetrasodium ethylenediaminetetraacetic acid, and glyceryl stearate and polyethylene glycol-100.

In an embodiment, the ellagic acid composition includes one or more exfoliants selected from salicylic acid.

In an embodiment, the ellagic acid composition includes one or more emulsifiers selected from glyceryl stearate and polyethylene glycol-100, stearic acid, polypropylene glycol-5-ceteth-20, and polyacrylamide and C13-14 isoparaffin and laureth-7.

In an embodiment, the ellagic acid composition include one or more cleansing agents selected from stearic acid.

In an embodiment, the ellagic acid composition includes one or more emulsion stabilizers selected from cetyl alcohol, xanthan gum, and acrylates/C10-30 alkyl acrylate crosspolymer.

In an embodiment, the ellagic acid composition includes one or more emollients selected from diisopropyl sebacate, octyldodecanol, dimethicone, and polyacrylamide and C13-14 isoparaffin and laureth-7.

In an embodiment, the ellagic acid composition includes one or more antioxidants selected from Ginkgo Biloba leaf extract, tocopherol, Glycyrrhiza Glabra root extract, and ellagic acid.

In an embodiment, the ellagic acid composition includes one or more binders selected from xanthan gum, and polyacrylamide and C13-14 isoparaffin and laureth-7.

In an embodiment, the ellagic acid composition includes one or more viscosity increasers selected from xanthan gum, and acrylates/C10-30 alkyl acrylate crosspolymer.

In an embodiment, the ellagic acid composition includes one or more denaturants selected from sodium hydroxide.

In an embodiment, the ellagic acid composition includes one or more pH adjusters selected from sodium hydroxide and potassium hydroxide.

In an embodiment, the ellagic acid composition includes one or more buffering agents selected from sodium hydroxide and potassium hydroxide.

In an embodiment, the ellagic acid composition includes one or more plant extracts selected from Ginkgo Biloba leaf extract, Rosa Centifolia flower extract, Glycyrrhiza Glabra root extract, and Mentha Piperita extract.

In an embodiment, the ellagic acid composition includes one or more algae extracts selected from Palmaria Palmata and Chlorella Vulgaris extract.

In an embodiment, the ellagic acid composition includes one or more seed extracts selected from Helianthus Annuus seed extract.

The following are representative components of an about 0.5% by weight ellagic acid composition (Ellagic Acid Formula 1) suitable for use with all the embodiments of current waveforms in iontophoresis treatments. The amounts are given in weight percent. The component names follow the International Nomenclature of Cosmetic Ingredients.
Water (a solvent): 15% (range of 5% to 25% and any value in between);
Glycerin (a humectant and skin protectant): 7% (range of 0% to 14% and any value in between);
Phenoxyethanol (a preservative): 0.5% (range of 0% to 1% and any value in between);
Propylene glycol (a humectant and skin conditioner): 10% (range of 0% to 20% and any value in between);
Methylparaben (a preservative): 0.15% (range of 0% to 1% and any value in between);
Tetrasodium ethylenediaminetetraacetic acid (a chelating agent): 0.1% (range of 0% to 1% and any value in between);
Ginkgo Biloba leaf extract (an antioxidant): 0.01% (range of 0% to 1% and any value in between);
Salicylic acid (an exfoliant and skin conditioner): 0.1% (range of 0% to 1% and any value in between);
Methyl gluceth-20 (a humectant and skin conditioner): 3% (range of 0% to 6% and any value in between);
Glyceryl Stearate and polyethylene glycol-100 stearate (an emulsifier and chelating agent): 1.45% (range of 0% to 3% and any value in between);
Stearic acid (a cleansing agent and emulsifying agent): 1% (range of 0% to 2% and any value in between);
Cetyl alcohol (a stabilizer): 0.5% (range of 0% to 1% and any value in between);
Diisopropyl sebacate ( an emollient): 4.5% (range of 0% to 9% and any value in between);
Ethylparaben (a preservative): 0.1% (range of 0% to 1% and any value in between);
Octyldodecanol (an emollient): 2% (range of 0% to 4% and any value in between);
Tocopherol (an antioxidant and skin conditioner): 0.1% (range of 0% to 1% and any value in between);
Fragrance: 0.15% (range of 0% to 1% and any value in between);
Fragrance: .005% (range of 0% to 1% and any value in between);
Dimethicone (an emollient and skin conditioner): 2.5% (range of 0% to 5% and any value in between);
Xanthan gum (a binder and stabilizer, viscosity increaser): 0.1% (range of 0% to 1% and any value in between);
Acrylates/C10-30 alkyl acrylate crosspolymer (a stabilizer and viscosity increaser): 0.15% (range of 0% to 1% and any value in between);
Water (a solvent): 1% (range of 0% to 2% and any value in between);
Sodium Hydroxide (a denaturant, pH adjuster, and buffering agent): 0.016% (range of 0% to 1% and any value in between);
Deionized water (a solvent): 42.894% (range of 20% to 50% and any value in between);
Rosa Centifolia flower extract (a skin conditioner): 0.1% (range of 0% to 1% and any value in between);
Hydrolyzed soy flour (a skin conditioner): 0.5% (range of 0% to 1% and any value in between);
Yeast extract (a skin conditioner): 0.1% (range of 0% to 1% and any value in between);
Glycyrrhiza Glabra (licorice) root extract (an antioxidant and humectant): 0.1% (range of 0% to 1% and any value in between);
Mentha Piperita (peppermint) extract (a skin conditioner): 0.05% (range of 0% to 1% and any value in between);
Helianthus Annuus (sunflower) seed extract (a skin conditioner): 0.5% (range of 0% to 1% and any value in between);
Deionized Water (a solvent): 5% (range of 0% to 10% and any value in between);
Polypropylene glycol-5-ceteth-20 (an emulsifier): 0.025% (range of 0% to 1% and any value in between);
Ellagic acid (an antioxidant and skin conditioner): 0.5% (range of 0.01% to 10% and any value in between);
Polyacrylamide and C13-14 isoparaffin and laureth-7 (a binder, emollient, and emulsifier): 0.8% (range of 0% to 2% and any value in between);
pH: 4 to 8 and any value in between.

The following are representative components of an about 0.5% by weight ellagic acid composition (Ellagic Acid Formula 2) suitable for use with all the embodiments of current waveforms in iontophoresis treatments. The amounts are given in weight percent. The component names follow the International Nomenclature of Cosmetic Ingredients.
Water (a solvent): 20% (range of 10% to 30% and any value in between);
Glycerin (a humectant and skin protectant): 7% (range of 0% to 14% and any value in between);
Phenoxyethanol (a preservative): 0.5% (range of 0% to 1% and any value in between);
Propylene glycol (a humectant and skin conditioner): 10% (range of 0% to 20% and any value in between);
Tetrasodium ethylenediaminetetraacetic acid (a chelating agent): 0.1% (range of 0% to 1% and any value in between);
Caprylyl glycol (a humectant and skin conditioner): 0.15% (range of 0% to 1% and any value in between);
Ginkgo Biloba leaf extract (an antioxidant): 0.01% (range of 0% to 1% and any value in between);
Salicylic acid (an exfoliant and skin conditioner): 0.2% (range of 0% to 1% and any value in between);
Methyl gluceth-20 (a humectant and skin conditioner): 3% (range of 0% to 6% and any value in between);
Glyceryl Stearate and polyethylene glycol-100 stearate (an emulsifier and chelating agent): 1.45% (range of 0% to 4% and any value in between);
Stearic acid (a cleansing agent and emulsifying agent): 1% (range of 0% to 2% and any value in between);
Cetyl alcohol (a stabilizer): 0.75% (range of 0% to 2% and any value in between);
Diisopropyl sebacate ( an emollient): 4.5% (range of 0% to 9% and any value in between);
Octyldodecanol (an emollient): 2% (range of 0% to 4% and any value in between);
Tocopherol (an antioxidant and skin conditioner): 0.1% (range of 0% to 1% and any value in between);
Fragrance: 0.15% (range of 0% to 1% and any value in between);
Fragrance: 0.005% (range of 0% to 1% and any value in between);
Dimethicone (an emollient and skin conditioner): 2.5% (range of 0% to 5% and any value in between);
Xanthan gum (a binder and stabilizer, viscosity increaser): 0.1% (range of 0% to 1% and any value in between);
Acrylates/C10-30 alkyl acrylate crosspolymer (a stabilizer and viscosity increaser): 0.2% (range of 0% to 1% and any value in between);
Sodium Hydroxide (a denaturant, pH adjuster, and buffering agent): 0.05% (range of 0% to 1% and any value in between);
Deionized water (a solvent): 36.61% (range of 20% to 50% and any value in between);
Rosa Centifolia flower extract (a skin conditioner): 0.1% (range of 0% to 1% and any value in between);
Hydrolyzed soy flour (a skin conditioner): 0.45% (range of 0% to 1% and any value in between);
Yeast extract (a skin conditioner): 0.1% (range of 0% to 1% and any value in between);
Glycyrrhiza Glabra (licorice) root extract (an antioxidant and humectant): 0.1% (range of 0% to 1% and any value in between);
Mentha Piperita (peppermint) extract (a skin conditioner): 0.05% (range of 0% to 1% and any value in between);
Helianthus Annuus (sunflower) seed extract (a skin conditioner): 0.5% (range of 0% to 1% and any value in between);
Palmaria Palmata extract (a skin protectant): 1% (range of 0% to 2% and any value in between);
Chlorella Vulgaris extract (a skin conditioner): 1% (range of 0% to 2% and any value in between);
Deionized Water (a solvent): 5% (range of 0% to 10% and any value in between);
Polypropylene glycol-5-ceteth-20 (an emulsifier): 0.025% (range of 0% to 1% and any value in between);
Ellagic acid (an antioxidant and skin conditioner): 0.5% (range of 0.01% to 10% and any value in between);
Polyacrylamide and C13-14 isoparaffin and laureth-7 (a binder, emollient, and emulsifier): 0.8% (range of 0% to 2% and any value in between);
pH: 4 to 8 and any value in between.

In an embodiment, an ellagic acid composition herein can "comprise" the specified components, leaving open the possibility of other unspecified components. In an embodiment, an ellagic acid composition herein can "consist" of the specific components, meaning the composition only includes the specified components. Stated another way, the specified components constitute 100% by weight of the ellagic acid composition.

### EXAMPLE

The behavior of ellagic acid (at 0.2%) formulated in a mixture of polar solvents was investigated for skin penetration using iontophoresis set to "positive galvanic current" (0.3 mA/cm2) at application times of 10 and 20 minutes.

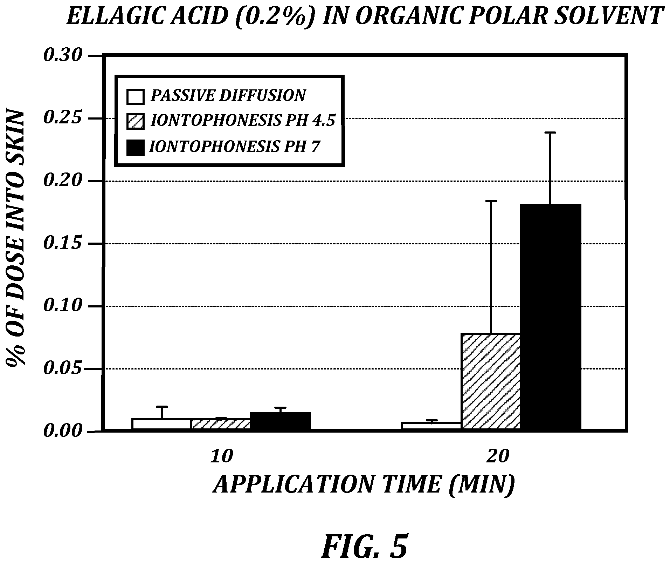

The results in FIGURE 5 show an improvement in penetration for both charged (iontophoresis pH 7.0) and uncharged forms (iontophoresis pH 4.5) compared to the control particularly at the 20 min treatment.

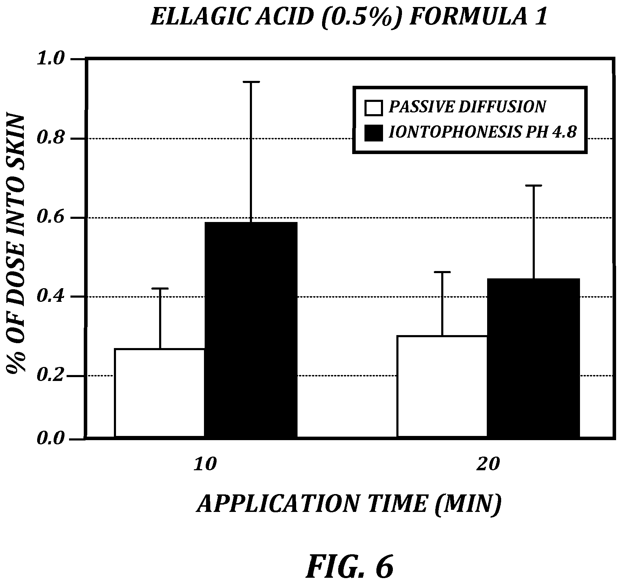

Interestingly, as shown in FIGURE 6, for the Ellagic Acid Formula 1, a 10 minute application showed higher delivery of ellagic acid compared to a 20 minute application.

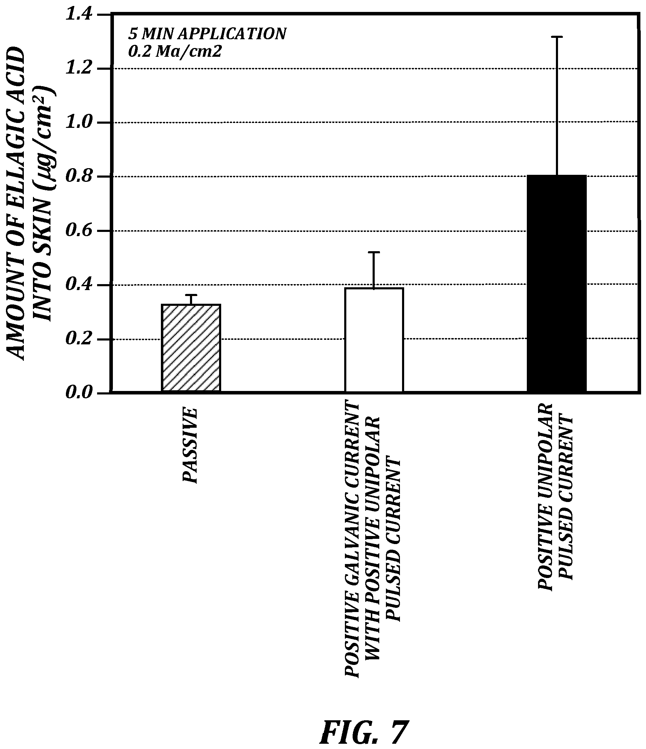

The passage of ellagic acid for Ellagic Acid Formula 2 containing water: organic solvents in a ratio of 4:1 (representing around 78% by weight relative to the total weight of the composition), after the application of different current modes "positive galvanic current" combined with "biphasic pulsed current" and/or "positive monophasic pulsed current", was then evaluated to determine the best penetration enhancement conditions at a "shorter application time" and "low current settings" (FIGURE 7).

Analysis of the skin penetration profiles suggested that positive monophasic pulsed applied during 5 min at 0.2 mA/cm2 improved the topical transport of ellagic acid compared to that obtained from its passive application. The method mainly consists on the facilitated absorption phase in which the penetration of ellagic acid, preferably in the uncharged form, is efficiently enhanced by iontophoresis treatment, most likely by electro-osmosis.

While illustrative embodiments have been illustrated and described, it will be appreciated that various changes can be made therein without departing from the spirit and scope of the invention.

## Claims

1. An iontophoresis device, comprising:
a. an electrode assembly including at least one electrode and an ellagic acid composition, the ellagic acid composition including one or more of ellagic acid, an ellagic acid derivative, an ion of ellagic acid, and an ion of an ellagic acid derivative; and
b. circuitry operably coupled to the electrode assembly and configured to concurrently generate at least a continuous direct current stimulus and a pulsed current stimulus at a first electrode, the continuous direct current stimulus and the pulsed current stimulus of a character and for a duration sufficient to transdermally deliver the ellagic acid composition to a biological subject.

2. The iontophoresis device of claim 1, wherein the electrode assembly includes at least one reservoir holding an ellagic acid composition comprising
a. one or more of ellagic acid, an ellagic acid derivative, an ion of ellagic acid, and an ion of an ellagic acid derivative present in amounts ranging from about 0.01% to about 30% by weight; and
b. a polar solvent present in an amount ranging from about 0.1 % by weight to about 99% by weight, and wherein the electrode assembly preferably includes at least one reservoir holding an ellagic acid composition further comprising one or more of
c. Acrylates/C10-30 alkyl acrylate crosspolymer present in an amount ranging from about 0% by weight to about 1% by weight;
d. Cetyl alcohol present in an amount ranging from about 0 % by weight to about 1% by weight;
e. Deionized water present in an amount ranging from about 20% by weight to about 60% by weight;
f. Diisopropyl sebacate present in an amount ranging from about 0 % by weight to about 9% by weight;
g. Dimethicone present in an amount ranging from about 0% by weight to about 5% by weight;
h. Ethylparaben present in an amount ranging from about 0% by weight to about 1% by weight;
i. Fragrance present in an amount ranging from about 0 % by weight to about 1% by weight;
j. Ginkgo Biloba extract present in an amount ranging from about 0% by weight to about 1% by weight;
k. Glycerin present in an amount ranging from about 0% by weight to about 14% by weight;
1. Glyceryl Stearate and polyethylene glycol-100 stearate present in an amount ranging from about 0% by weight to about 3% by weight;
m. Glycyrrhiza Glabra root extract present in an amount ranging from about 0% by weight to about 1% by weight;
n. Helianthus Annuus seed extract present in an amount ranging from about 0% by weight to about 1% by weight;
o. Hydrolyzed soy flour present in an amount ranging from about 0% by weight to about 1% by weight;
p. Mentha Piperita extract present in an amount ranging from about 0% by weight to about 1% by weight;
q. Methyl gluceth-20 present in an amount ranging from about 0% by weight to about 6% by weight;
r. Methylparaben present in an amount ranging from about 0% by weight to about 1% by weight;
s. Octyldodecanol present in an amount ranging from about 0% by weight to about 4% by weight;
t. Phenoxyethanol present in an amount ranging from about 0% by weight to about 1% by weight;
u. Polyacrylamide and C13-14 isoparaffin and laureth-7 present in an amount ranging from about 0% by weight to about 2% by weight;
v. Polypropylene glycol-5-ceteth-20 present in an amount ranging from about 0% by weight to about 1 % by weight;
w. Propylene glycol present in an amount ranging from about 0% by weight to about 20% by weight;
x. Rosa Centifolia flower extract present in an amount ranging from about 0% by weight to about 1% by weight;
y. Salicylic acid present in an amount ranging from about 0% by weight to about 1% by weight;
z. Sodium Hydroxide present in an amount ranging from about 0% by weight to about 1% by weight;
aa. Stearic acid present in an amount ranging from about 0% by weight to about 2% by weight;
bb. Tetrasodium ethylenediaminetetraacetic acid present in an amount ranging from about 0% by weight to about 1% by weight;
cc. Tocopherol present in an amount ranging from about 0% by weight to about 1% by weight;
dd. Xanthan gum present in an amount ranging from about 0% by weight to about 1% by weight; and
ee. Yeast extract present in an amount ranging from about 0% by weight to about 1% by weight.

3. The iontophoresis device of claim 1, wherein the continuous direct current stimulus comprises an average current density raging from about 0.01 mA/cm² to about 0.5 mA/cm², and wherein the continuous direct current stimulus preferably comprises an average current density of about 0.2 mA/cm².

4. The iontophoresis device of claim 1, wherein the pulsed current stimulus comprises an average current density raging from about 0.01 mA/cm² to about 10 mA/cm²; a pulse width ranging from about 50 microseconds to about 100 milliseconds; and a pulse frequency ranging from about 1 Hertz to about 2000 Hertz; and the duty cycle of pulses ranging from about 1% to about 90%, orwherein the pulsed current stimulus comprises an average current density raging from about 0.01 mA/cm² to about 10 mA/cm²; a pulse width ranging from about 50 microseconds to about 100 milliseconds; at least one pulse train having from 2 to 20 pulses; a pulse frequency ranging from about 0.1 Hertz to about 500 Hertz; and a duty cycle ranging from 1% to 90%, or wherein the pulsed current stimulus comprises an average current density raging from about 0.01 mA/cm² to about 10 mA/cm²; a pulse width ranging from about 50 microseconds to about 100 milliseconds; and at least one pulse train of about 2 to about 20 pulses with alternating polarity; a pulse frequency ranging from about 0.1 Hertz to about 500 Hertz; and a duty cycle of pulses ranging from about 1% to about 90%, or wherein the pulsed current stimulus comprises an amplitude current density of about 0.2 mA/cm²; an alternating pulse duration of about 500 microseconds; and a pulse frequency of about 200 Hertz.

5. The iontophoresis device of claim 1, wherein the electrode assembly includes at least one reservoir holding an ellagic acid composition.

6. The iontophoresis device of claim 1, wherein the electrode assembly includes at least one active electrode electrically coupled to a reservoir holding an ellagic acid composition; the electrode assembly operable to transdermally deliver the ellagic acid composition to a biological subject responsive to one or more inputs from the circuitry configured to concurrently generate the continuous direct current stimulus and the pulsed current stimulus.

7. The iontophoresis device of claim 1, wherein the electrode assembly is electrically coupled to at least one power source.

8. The iontophoresis device of claim 1, wherein the electrode assembly includes a least one active electrode assembly and at least one counter electrode assembly.

9. An iontophoresis assembly, comprising:
a. a current waveform generator with circuitry configured to
b. apply direct current at a current density of at most 0.5 mA/cm2 for up to 30 minutes, or
c. apply a pulse waveform current density at an amplitude of at most 1 mA/cm² for up to 30 minutes, or
d. apply concurrently a direct current waveform and a pulse waveform at an average current density of at most 0.5 mA/cm2 for up to 30 minutes, or a combination thereof in sequential with or without period of pause of up to 120 minutes, wherein the pulse waveform preferably includes monophasic pulses having a constant slope between a maximum and a minimum amplitude, or includes biphasic pulses having a constant slope between a maximum amplitude in a first current direction and a maximum amplitude in a second current direction the assembly preferably further comprising an ellagic acid composition within the device, wherein the ellagic composition comprises: from 0.01 % by weight to 30% by weight ellagic acid or ellagic acid derivatives or their ions, and from 0.1 % by weight to 99% by weight of organic polar solvents, including water.

10. An iontophoresis method, comprising:
a. concurrently delivering a continuous direct current and a pulsed current to a biological subject, the continuous direct current and the pulsed current of a character and for a duration sufficient to transdermally deliver an ellagic acid composition including at least one of ellagic acid, an ellagic acid derivative, an ion of ellagic acid, an ion of an ellagic acid derivative, or mixtures thereof, wherein concurrently delivering the continuous direct current and the pulsed current to a biological subject preferably includes generating a continuous direct current stimulus having an average current density raging from about 0.01 mA/cm² to about 0.5 mA/cm², or wherein concurrently delivering the continuous direct current and the pulsed current to a biological subject preferably includes generating a continuous direct current stimulus having an average current density of about 0.2 mA/cm², or wherein concurrently delivering the continuous direct current and the pulsed current to a biological subject preferably includes generating a pulsed current stimulus having an amplitude current density raging from about 0.01 mA/cm² to about 10 mA/cm²; a pulse duration ranging from about 50 microseconds to about 100 milliseconds; and a pulse frequency ranging from about 0.1 Hertz to about 500 Hertz; and a duty cycle ranging from 1% to 90%, or wherein concurrently delivering the continuous direct current having an average current density of about 0.2 mA/cm² and the pulsed current to a biological subject preferably includes generating a pulsed alternating current stimulus having an amplitude current density of about 0.2 mA/cm2; a pulse duration of about 500 microseconds; and a pulse frequency of about 200 Hertz or wherein the pulsed current stimulus preferably comprises an average current density raging from about 0.01 mA/cm² to about 10 mA/cm²; a pulse width ranging from about 50 microseconds to about 100 milliseconds; at least one pulse train having from about 2 to about 20 pulses; a pulse frequency ranging from about 0.1 Hertz to about 500 Hertz; and a duty ranging from 1% to 90% or wherein the pulsed current stimulus preferably comprises an average current density raging from about 0.01 mA/cm² to about 10 mA/cm²; a pulse width ranging from about 50 microseconds to about 100 milliseconds; at least one pulse train having from about 2 to 20 pulses with alternating polarity; a pulse frequency ranging from about 0.1 Hertz to about 500 Hertz; and a duty cycle ranging from 1% to 90%,
b. wherein concurrently delivering the continuous direct current and the pulsed current to a biological subject preferably includes generation a pulsed current having sinusoidal waveforms, non-sinusoidal waveforms, or combinations thereof, or wherein concurrently delivering the continuous direct current and the pulsed current to a biological subject preferably includes generation a pulsed current having periodic square waveforms, rectangular waveforms, saw tooth waveforms, spiked waveforms, trapezoidal waveforms, triangle waveforms, exponential decay, or combinations thereof, ,
c. the iontophoresis method preferably further comprising:
d. transdermally delivering an ellagic acid composition including
e. one or more of ellagic acid, an ellagic acid derivative, an ion of ellagic acid, and an ion of an ellagic acid derivative present in amounts ranging from about 0.01% to about 30% by weight; and
f. a polar solvent present in an amount ranging from about 0.1 % by weight to about 99% by weight.

11. An iontophoresis composition, comprising:
a. one or more of ellagic acid, an ellagic acid derivative, an ion of ellagic acid, and an ion of an ellagic acid derivative present in amounts ranging from about 0.01% to about 30% by weight; and
b. a polar solvent present in an amount ranging from about 0.1 % by weight to about 99% by weight.

12. The iontophoresis composition of claim 11, further comprising:
a. one or more anionic, cationic, nonionic, or amphoteric polymers in an amount ranging from about 0.01 % by weight to about 10%;
b. one or more anionic, cationic, nonionic, or amphoteric surfactants in an amount preferably ranging from about 0.01 % by weight to about 30%;
c. one or more penetrating agents in an amount ranging from 0.1 % by weight to about 30%;
d. one or more preservatives agents in an amount ranging from 0.01% % by weight to about 5 %;
e. one or more buffering agents in an amount ranging from 0.01% % by weight to about 10%;
f. one or more exfoliating agents in an amount ranging from 0.1% % by weight to about 10%.

13. The iontophoresis composition of claim 11, further comprising:
a. Deionized water present in an amount ranging from about 20% by weight to about 60% by weight;
b. Propylene glycol present in an amount ranging from about 0% by weight to about 20% by weight; and
c. Sodium Hydroxide present in an amount ranging from about 0% by weight to about 1% by weight.

14. The iontophoresis composition of claim 13, further comprising:
a. Acrylates/C10-30 alkyl acrylate cross polymer present in an amount ranging from about 0% by weight to about 1% by weight;
b. Cetyl alcohol present in an amount ranging from about 0 % by weight to about 1% by weight;
c. Diisopropyl sebacate present in an amount ranging from about 0 % by weight to about 9% by weight;
d. Dimethicone present in an amount ranging from about 0% by weight to about 5% by weight;
e. Ethylparaben present in an amount ranging from about 0% by weight to about 1% by weight;
f. Fragrance present in an amount ranging from about 0 % by weight to about 1% by weight;
g. Ginkgo Biloba extract present in an amount ranging from about 0% by weight to about 1% by weight;
h. Glycerin present in an amount ranging from about 0% by weight to about 14% by weight;
i. Glyceryl Stearate and polyethylene glycol-100 stearate present in an amount ranging from about 0% by weight to about 3% by weight;
j. Glycyrrhiza Glabra root extract present in an amount ranging from about 0% by weight to about 1% by weight;
k. Helianthus Annuus seed extract present in an amount ranging from about 0% by weight to about 1% by weight;
l. Hydrolyzed soy flour present in an amount ranging from about 0% by weight to about 1% by weight;
m. Mentha Piperita extract present in an amount ranging from about 0% by weight to about 1% by weight;
n. Methyl gluceth-20 present in an amount ranging from about 0% by weight to about 6% by weight;
o. Methylparaben present in an amount ranging from about 0% by weight to about 1% by weight;
p. Octyldodecanol present in an amount ranging from about 0% by weight to about 4% by weight;
q. Phenoxyethanol present in an amount ranging from about 0% by weight to about 1% by weight;
r. Polyacrylamide and C13-14 isoparaffin and laureth-7 present in an amount ranging from about 0% by weight to about 2% by weight;
s. Polypropylene glycol-5-ceteth-20 present in an amount ranging from about 0% by weight to about 1 % by weight;
t. Rosa Centifolia flower extract present in an amount ranging from about 0% by weight to about 1% by weight;
u. Salicylic acid present in an amount ranging from about 0% by weight to about 1% by weight;
v. Stearic acid present in an amount ranging from about 0% by weight to about 2% by weight;
w. Tetrasodium ethylenediaminetetraacetic acid present in an amount ranging from about 0% by weight to about 1% by weight;
x. Tocopherol present in an amount ranging from about 0% by weight to about 1% by weight;
y. Xanthan gum present in an amount ranging from about 0% by weight to about 1% by weight; and
z. Yeast extract present in an amount ranging from about 0% by weight to about 1% by weight.

15. The iontophoresis composition of claim 11, further comprising:
a pH ranging from about 4 to about 8.
